# EUROPEAN PATENT SPECIFICATION

(11) **EP 3 825 683 B1**
(45) Date of publication and mention of the grant of the patent: **29.01.2025**
(21) Application number: 20214733.6
(22) Date of filing: 28.06.2018
(51) Int. Cl.: G01N 27/22

(54) **SYSTEM AND METHOD FOR ANALYTE SENSING IN PHYSIOLOGICAL GAS SAMPLES**
SYSTEM UND VERFAHREN FÜR ANALYTNACHWEIS IN PHYSIOLOGISCHEN GASPROBEN
SYSTÈME ET PROCÉDÉ DE DÉTECTION D'ANALYTES DANS DES ÉCHANTILLONS DE GAZ PHYSIOLOGIQUE

(30) Priority: 18.07.2017 US 201762533916 P; 06.09.2017 NL 2019492
(43) Date of publication of application: 26.05.2021
(62) Divisional of application: 18180455.0
(73) Proprietor: Regents of the University of Minnesota, Minneapolis, MN 55455-2020 (US)
(72) Inventor: KELLY, David W., Arden Hills, MN Minnesota 55112 (US); SHERWOOD, Gregory J., Arden Hills, MN Minnesota 55112 (US); NELSON, Justin Theodore, Arden Hills, MN Minnesota 55112 (US)
(74) Representative: Vossius & Partner Patentanwälte Rechtsanwälte mbB

(56) References cited:
- EP-A1- 3 093 653
- EP-A2- 2 542 921
- EP-B1- 2 542 921
- EP-B1- 3 093 653
- US-A1- 2012 306 802
- US-A1- 2013 100 067
- MA RUI ET AL: "Acetone sensing using graphene quantum capacitance varactors", 2016 IEEE SENSORS, IEEE, 30 October 2016 (2016-10-30), pages 1 - 3, XP033036953, DOI: 10.1109/ICSENS.2016.7808671
- DEEN DAVID A ET AL: "Graphene-Based Quantum Capacitance Wireless Vapor Sensors", IEEE SENSORS JOURNAL, IEEE SERVICE CENTER, NEW YORK, NY, US, vol. 14, no. 5, 1 May 2014 (2014-05-01), pages 1459 - 1466, XP011543455, ISSN: 1530-437X, [retrieved on 20140318], DOI: 10.1109/JSEN.2013.2295302
- A. OPREA ET AL: "Integrated Temperature, Humidity and Gas Sensors on Flexible Substrates for Low-Power Applications", SENSORS, 2007 IEEE, 1 January 2007 (2007-01-01), Pi, pages 158 - 161, XP055466331, ISBN: 978-1-4244-1261-7, DOI: 10.1109/ICSENS.2007.4388360
- ZHANG XU ET AL: "A Wide Measurement Range and Fast Update Rate Integrated Interface for Capacitive Sensors Array", IEEE TRANSACTIONS ON CIRCUITS AND SYSTEMS I: REGULAR PAPERS, IEEE, US, vol. 61, no. 1, 1 January 2014 (2014-01-01), pages 2 - 11, XP011536831, ISSN: 1549-8328, [retrieved on 20140106], DOI: 10.1109/TCSI.2013.2264696

## Description

The present disclosure invention relates to systems, devices and methods for analyte sensing in physiological gas samples. More specifically, the present invention disclosure relates to systems, devices and methods for measuring analyte presence on graphene varactors.

In the process of providing health care, clinicians often make physical observations and run tests to gather data about a patient. After collecting data and analyzing other aspects, such as a given patient's health history, the clinician often forms a diagnosis and then selects a therapy to treat the diagnosed condition.

Knowledge of the presence and/or concentrations of various chemical analytes, such as volatile organic compounds, can be extremely useful in forming a diagnosis.

MA RUI ET AL: "Acetone sensing using graphene quantum capacitance varactors", 2016 IEEE SENSORS, IEEE, (20161030), doi:10.1109/ICSENS.2016.7808671, pages 1 - 3, relates to sensing of acetone using the quantum capacitance in graphene.

DEEN DAVID A ET AL: "Graphene-Based Quantum Capacitance Wireless Vapor Sensors", IEEE SENSORS JOURNAL, IEEE SERVICE CENTER, NEW YORK, NY, US, vol. 14, no. 5, doi:10.1109/JSEN.2013.2295302, ISSN 1530-437X, relates to a wireless vapor sensor based on the quantum capacitance effect in graphene.

However, measuring such chemical analytes in a clinically practical manner remains technically challenging.

The present invention relates to systems, devices and methods for analyte sensing in physiological gas samples as recited in the independent claims. Embodiments of the invention are recited in the dependent claims.

A system for measuring the presence of a plurality of different analytes on a plurality of graphene varactors according to the present invention includes a capacitance to digital converter (CDC) and a plurality of graphene varactors.

The CDC is in electrical communication with the graphene varactors and is configured to measure the capacitance of the graphene varactors in response to an excitation signal over a plurality of DC bias voltages.

The system further includes a multiplexor configured to selectively provide electrical communication between the CDC and the plurality of graphene varactors.

An excitation signal is applied to a selected graphene varactor through the multiplexor.

The excitation signal can include an alternating voltage component.

The amplitude of the excitation signal can be fixed by the CDC.

A controller circuit is included and is configured to receive an electrical signal reflecting the capacitance of the graphene varactors.

The controller circuit can include a microcontroller.

The system can further include an electronic component in electrical communication with the controller circuit and the CDC, the electronic component selected from the group consisting of an I2C or an SPI.

The graphene varactors can include a functionalized graphene varactor.

The controller circuit can be configured to calculate at least one parameter for the graphene varactor selected from the group consisting of:
- maximum slope of capacitance to voltage;
- change in maximum slope of capacitance to voltage over a baseline value;
- minimum slope of capacitance to voltage;
- change in minimum slope of capacitance to voltage over a baseline value;
- minimum capacitance;
- change in minimum capacitance over a baseline value;
- voltage at minimum capacitance;
- change in voltage at minimum capacitance;
- maximum capacitance;
- change in maximum capacitance;
- ratio of maximum capacitance to minimum capacitance;
- response time constant; and
- ratios of any of the foregoing between the graphene varactor and a second graphene varactor.

The graphene varactor(s) can be grounded.

The graphene varactor(s) can be in electrical communication with an independent excitation output of the CDC.

The system can further include one or more programmable digital to analog converters (DACs) in series with the plurality of graphene sensors.

The system can further include a switch, the CDC in electrical communication with the switch and controlling the switch to selectively provide communication with output voltages of two programmable digital to analog converters (DACs), the programmed voltage difference between the DACs determining the excitation amplitude, and the difference between the programmed average voltage of the DACs and the bias at the CDC input determining the DC bias.

The invention also relates to a gas sampling device, for example a hand-held breath sampling device or a gas sampling device for use with catheters and/or endoscopy systems, for sensing gaseous analytes for example volatile organic compounds, the device comprising or incorporated into the above referred to system.

The invention also relates to the use of this system and/or gas sampling device for determining the presence of and/or measuring a chemical analyte such as a volatile organic compound, in particular for use in the following method according to the present invention.

The invention also relates to a method for measuring analyte presence on a graphene varactor. The method includes measuring a capacitance of the graphene varactor over a range of DC bias voltages; comparing the measured capacitance at one or more DC bias voltages to one or more corresponding baseline capacitance values; and determining the presence or absence of an analyte based on the comparison.

The method can further include determining one or more aspects selected from the group consisting of:
- maximum slope of capacitance to voltage;
- change in maximum slope of capacitance to voltage over a baseline value;
- minimum slope of capacitance to voltage;
- change in minimum slope of capacitance to voltage over a baseline value;
- minimum capacitance;
- change in minimum capacitance over a baseline value;
- voltage at minimum capacitance;
- change in voltage at minimum capacitance;
- maximum capacitance;
- change in maximum capacitance;
- ratio of maximum capacitance to minimum capacitance;
- response time constant; and
- ratios of any of the foregoing between two different graphene sensors.

The method can further include forming a capacitance to voltage curve from the measured capacitance of the graphene varactor over the range of DC bias voltages.

The method can further include comparing the formed capacitance to voltage curve with a baseline capacitance to voltage curve.

The method can further include distinguishing between different analytes binding to the graphene varactor.

The range of DC bias voltages can be in the following ranges -3 V to 3 V; -2.5 V to 2.5 V; -2 V to 2 V; - 1.5 V to 1.5 V; - 1 V to 1 V; -0.5 V to 0.5 V.

This summary is an overview of some of the teachings of the present application and is not intended to be an exclusive or exhaustive treatment of the present subject matter. Further details are found in the detailed description and appended clauses. Other aspects will be apparent to persons skilled in the art upon reading and understanding the following detailed description and viewing the drawings that form a part thereof, each of which is not to be taken in a limiting sense. The scope herein is defined by the appended clauses and their legal equivalents.

Aspects may be more completely understood in connection with the following drawings, in which:
- FIG. 1 is a schematic view of various components of a system in accordance with the present invention disclosure.
- FIG. 2 is a schematic exploded view of a graphene varactor in accordance with the present invention disclosure.
- FIG. 3 is a schematic cross-sectional view of a graphene varactor in accordance with the present invention disclosure.
- FIG. 4 is a schematic cross-sectional view of elements of a gas sensing device consistent with the technology disclosed herein.
- FIG. 5 is a schematic diagram of circuitry to measure the capacitance of a plurality of graphene sensors in accordance with the present invention disclosure.
- FIG. 6 is a schematic diagram of circuitry to measure the capacitance of a plurality of graphene sensors in accordance with the present invention disclosure.
- FIG. 7 is a schematic diagram of circuitry to measure the capacitance of a plurality of graphene sensors in accordance with the present invention disclosure.
- FIG. 8 is a graph showing capacitance versus DC bias voltage for a graphene varactor in accordance with the present invention disclosure.
- FIG. 9 is a graph showing capacitance versus DC bias voltage for a graphene varactor in accordance the present invention disclosure.
- FIG. 10 is a graph showing capacitance versus DC bias voltage for a functionalized graphene varactor in response to acetone binding in accordance with the present invention disclosure.
- FIG. 11 is a graph showing capacitance versus DC bias voltage for a functionalized graphene varactor in response to octane binding in accordance with the present invention disclosure.
- FIG. 12 is a graph showing capacitance versus DC bias voltage for a functionalized graphene varactor in response to octanol binding in accordance with the present invention disclosure.

Volatile organic compounds, as sensed in breath samples or other gas samples, can provide valuable information about the health status of a patient. In particular, patterns of volatile organic compounds (including the presence, absence, and/or concentration of a plurality of different volatile organic compounds) in a breath or gas sample of a patient can be associated with various disease states and/or particular health statuses. The disease states can include, but are not limited to, lung cancer, colon cancer, pulmonary disease (e.g. asthma, COPD), cardiovascular disease (e.g. heart failure), infectious diseases, digestive and inflammatory diseases (e.g. inflammatory bowel diseases such as Crohn's, colitis, or the like) or diabetes.

Graphene varactors are one type of device that can be used to measure volatile organic compounds. Various properties of graphene varactors, such as capacitance, can change in response to the presence of volatile organic compounds thereon. This has been used to propose sensors in which a value of the capacitance, measured at a previously determined advantageous bias voltage, is measured and is correlated with the concentration of a specific analyte However, measurement of properties of the graphene varactors, such as capacitance, remains technically challenging. Passive wireless techniques can be used, but may not be sufficiently accurate or fast for all purposes.

However, the present invention disclosure can be used to rapidly and accurately measure the capacitance of a plurality of graphene varactors due to the use of a capacitance-to-digital converted (CDC). Existing systems tend to rely on other measurement circuits, which are more commonly used and may be more advantageous for measuring capacitance at a given (single) bias voltage. However, a CDC component enables a rapid sweep across bias voltages in a manner that makes it practical to rapidly collect data for a plurality of DC bias voltages.

Furthermore, an advantage of using a CDC may be that the density of sensors on a substrate that can be read in an inexpensive and portable system may be significantly increased.

Therefore, a CDC allows for sensors which are more precise and which may be used to accurately and quickly detect various different analytes in a same sample, leading to a large and rich data set which can provide information that would be difficult or even impossible to derive using existing systems.

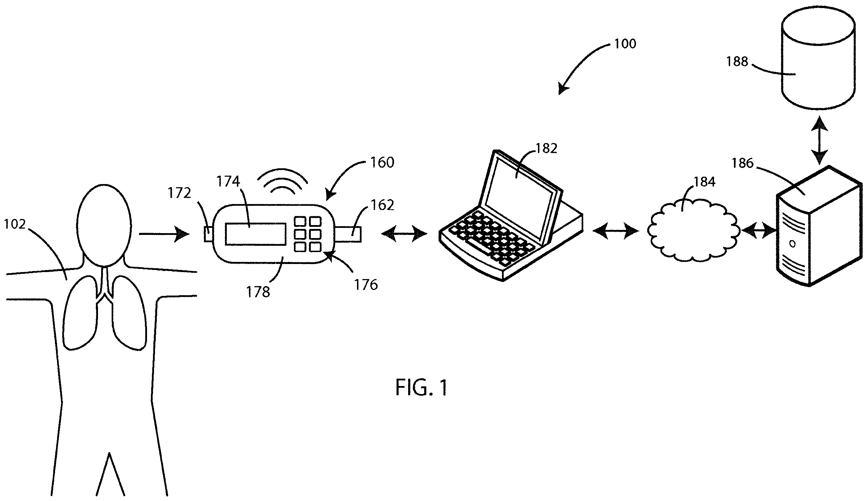

Referring now to FIG. 1, a schematic view is shown of possible components of a system 100 in accordance with the present invention disclosure. The system 100 can include a breath sensing device 160 for sensing gaseous analytes (or volatile organic compounds). In this figure, the system is exhibited in a hand-held format. It will be appreciated, however, that many other formats for the system are contemplated herein.

The breath sensing device 160 can include a housing 178. The breath sensing device 160 can include a mouthpiece 162 into which a subject to be evaluated can blow a breath sample. The breath sensing device 160 can also include a display screen 174 and a user input device 176, such as a keyboard. The breath sensing device 160 can also include a gas outflow port 172. Aspects of breath sensing systems and devices are described in U.S. Publ. Appl. No. 2016/0109440. While FIG. 1 shows a breath sensing device, it will be appreciated that other types of gas sampling systems can also be used herein. For example, gas sampling devices for use with catheters and endoscopy systems can also be used. An exemplary gas sampling device in the context of a catheter or endoscopy device is described in U.S. Appl. No. 62/350,345.

The system 100 can include a local computing device 182 that can include a microprocessor, input and output circuits, input devices, a visual display, a user interface, and the like. The breath sensing device 160 can communicate with the local computing device 182 in order to exchange data between the breath sensing device 160 and the local computing device 182. The local computing device 182 can be configured to perform various processing steps with the data received from the breath sensing device 160, including, but not limited to, calculating various parameters described herein. However, it should be appreciated that the features associated with the local computing device 182 can be integrated into the breath sensing device 160. The local computing device 182 can be a laptop computer, a desktop computer, a server (real or virtual), a purpose dedicated computer device, or a portable computing device (including, but not limited to, a mobile phone, tablet, wearable device, etc.).

The local computing device 182 and/or the breath sensing device 160 can communicate with computing devices in remote locations through a data network 184, such as the Internet or another network for the exchange of data as packets, frames, or otherwise.

The system 100 can also include a computing device such as a server 186 (real or virtual). The server 186 can be located remotely from the breath sensing device 160. The server 186 can be in data communication with a database 188.

The database 188 can be used to store various patient information, such as that described herein. The database can specifically include an electronic medical database containing data regarding the health status of a patient, patterns of data associated with various conditions (such as that generated from machine learning analysis of large sets of patient data), demographic data and the like.

Graphene varactors can be prepared in various ways and with various geometries.

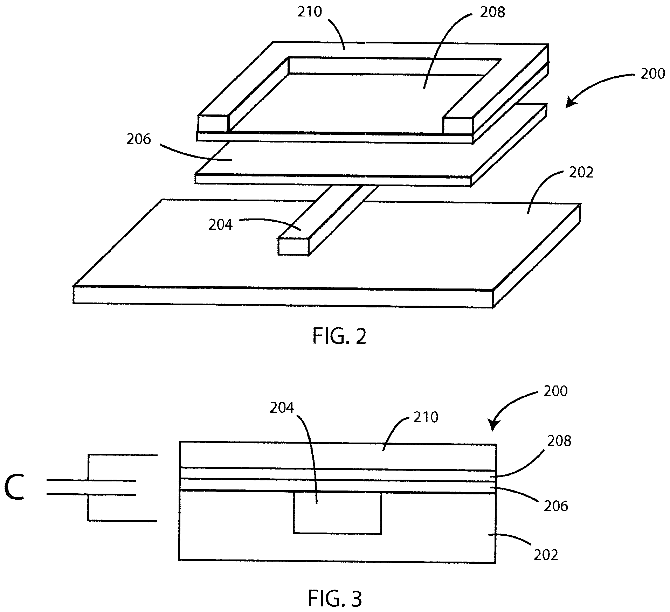

Referring now to FIG. 2, an exploded view of a graphene varactor 200 is shown. The graphene varactor 200 can include an insulator layer 202, a gate electrode 204, a dielectric layer 206, a graphene layer 208, and a contact electrode 210.

FIG. 3 shows a cross-sectional view of the graphene varactor 200. As is shown, the gate electrode 204 can be recessed into the insulator layer 202. The gate electrode 204 can be formed by etching a depression into the insulator layer 202 and then depositing an electrically conductive material in the depression to form the gate electrode 204. The insulator layer 202 can include various materials. The insulator layer 202 can be silicon dioxide formed on a silicon substrate (wafer). The dielectric layer 206 can be formed on a surface of the insulator layer 202 and the gate electrode 204. The dielectric layer 206 can be formed of a material, such as, silicon dioxide, aluminum oxide, hafnium dioxide, zirconium dioxide, hafnium silicate or zirconium silicate. The graphene layer 208 can be disposed on the dielectric layer 206. The graphene layer 208 can be a graphene monolayer. A contact electrode 210 can also be disposed on the surface (or a portion thereof) of the graphene layer 208. Aspects of exemplary graphene varactors can be found in U.S. Publ. App. No. 2014/0145735.

Further aspects of gas and/or breath sampling systems are described in U.S. Publ. Appl. No. 2016/0109440.

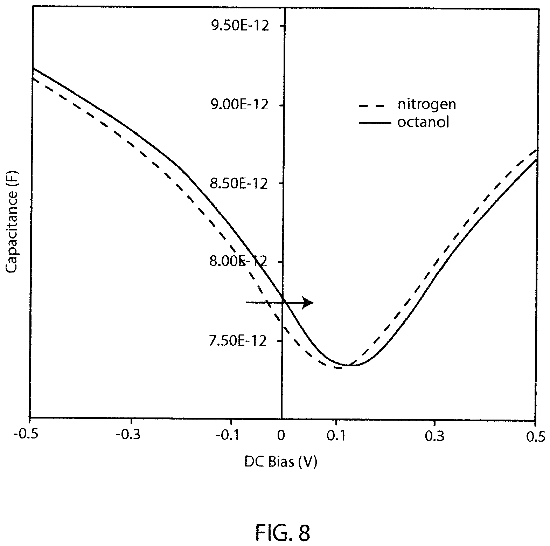

The graphene layer can be functionalized to make the graphene sensor specific for a particular analyte or class of analytes. As analytes bind to the graphene layer, the capacitance of the sensor changes. Further, as shown below with regard to FIGS. 8-9, the capacitance changes differently based on the voltage of the excitation current.

Systems herein can also include a breath and/or gas sensing device or system. In particular, systems herein can gather data on the presence, absence, and/or amount of various gaseous analytes including, but not limited to, volatile organic compounds.

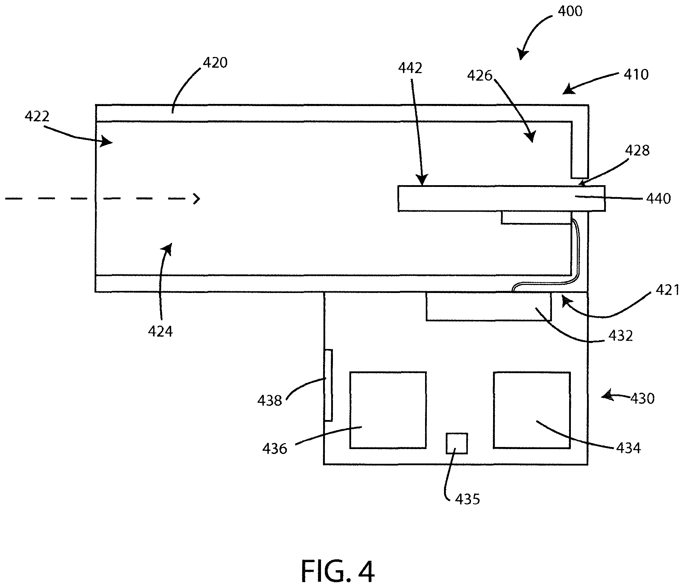

FIG. 4 is a schematic cross-sectional view of an example system 400 consistent with the technology disclosed herein. It will be appreciated that this schematic view has been simplified for ease of illustration and that embodiments of systems and devices herein can include various features not shown in FIG. 4. In addition, some embodiments of systems and devices herein may lack various features shown in FIG. 4. The system 400 is generally configured for collecting a gas sample and communicating data associated with the gas sample. The system 400 has a gas sampling device 410 and a docking station 430.

The gas sampling device 410 can be configured to collect a gas sample and facilitate testing of the gas sample to generate data. In some embodiments, the gas sampling device 410 can be configured as a handheld device. In such cases, the gas sampling device can be configured to be held in the hand of a care provider, a patient, or both, during certain steps of its use, while also being configured to be held or otherwise positioned in association with the docking station 430 during certain steps of its use.

The gas sampling device 410 can be configured to receive a gas sample, such as exhaled breath, from a patient and direct the gas sample to a testing location. The gas sampling device 410 generally has a housing 420 defining an airflow aperture 422, a gas testing chamber 426, a sensor receptacle 428, an airflow pathway 424, and a docking structure 421.

When receiving a gas sample, the gas (such as breath from a patient), can pass into the gas sampling device 410 through the airflow aperture 422, through the airflow pathway 424, into the gas testing chamber 426 and into contact with one or more measurement zones 442 of a disposable sensor test strip 440, and then out the end of the gas testing chamber 426 through the sensor receptacle 428, or through a separate exhaust port (not shown in this view). While this view depicts contact in some areas between the sensor receptacle 428 and the disposable sensor test strip 440, it will appreciated that there can be segments or areas where the sensor receptacle 428 and the disposable sensor test strip 440 do not contact or do not create sealing contact, thus allowing for a path for the gas to flow out through the sensor receptacle 428.

While FIG. 4 shows the airflow pathway 424 to be approximately the same size as the interior space of the housing 420, it will be appreciated that this is simply for ease of illustration and that the size of the airflow pathway 424 can be, in many cases, much smaller than the entire interior size of the housing 420, allowing for room for other components within the interior of the housing 420, such as other components described herein including, but not limited to, sensors, a power source, processing devices, communication hardware, conditioning elements, and the like.

The housing 420 can be constructed of a variety of materials and combinations of materials. The housing 420 can be a single cohesive structure or can be constructed of multiple components that are coupled to form the housing 420. As an illustrative example, a portion of the housing 420 that defines the airflow pathway 424 can be coupled to the portion of the housing 420 that defines the airflow aperture 422. The portion of the housing 420 that defines the airflow pathway 424 can include a conduit or tube with various different cross-sectional sizes and shapes. The conduit or tube can be formed from various materials including, but not limited to, polymers, metals, ceramics, glass, composites or the like. **In** some embodiments, surfaces lining the airflow pathway 424 can be coated with materials to provide various desirable functional properties.

The airflow aperture 422 is generally configured to provide an input for the gas sample at the housing 420. The airflow aperture 422 can be configured to be in fluid communication with a patient's mouth, although in some other embodiments a protective liner can be used to provide a barrier between the patient's mouth and the housing, which will be described in more detail, below.

The airflow pathway 424 generally is configured to direct the gas input at the airflow aperture 422 to the gas testing chamber 426. As such, the airflow pathway 424 generally extends from the airflow aperture 422 to the gas testing chamber 426. The airflow pathway 424 can have a cross-sectional area that is substantially the same along the length of the airflow pathway or it can vary. The gas testing chamber 426 can have different interior dimensions (e.g., height, width, etc.) than the airflow pathway leading to it.

The gas testing chamber 426 defines a testing location for the gas sample. The gas testing chamber 426 can be configured to receive a measurement zone 442 of a disposable sensor test strip 440. Accordingly, the sensor receptacle 428 defined by the housing 420 is generally configured to removably retain the disposable sensor test strip 440 within the gas testing chamber 426. The sensor receptacle 428 can be configured to slidably receive the disposable sensor test strip 440 that is manually inserted by a user. The disposable sensor test strip 440 can be inserted with its long (or major) axis parallel to the long (or major) axis of the housing 420. However, the disposable sensor test strip 440 can also be inserted with its long (or major) axis positioned differently with respect to the long (or major) axis of the housing 420, such as perpendicular. Example sensor test strips will be described in more detail, below.

While FIG. 4 depicts the test strip located approximately in the middle of the gas sampling device 410 (top to bottom with regard to the perspective of the figure), it will be appreciated that the test strip can be positioned biased toward the top or the bottom, to be closer to an exterior surface of the housing 420 or gas sampling device 410. The disposable sensor strip can be positioned less than 5 cm, 4 cm, 3 cm, 2 cm, 1 cm, 0.5 cm, 0.2 cm or less from exterior surface (or exterior wall) of the housing 420.

The docking station 430 is generally configured to collect data generated from testing the gas sample. The docking station 430 has a reading device 432 that can include circuitry to measure the capacitance of a plurality of graphene sensors. Aspects of circuitry to measure the capacitance of the graphene sensors are described in greater detail below. However, it will be appreciated that such circuitry can all be disposed in the gas sampling device 410, can all be disposed in the docking station 430, or can be distributed with some components in the gas sampling device 410 and some components in the docking station 430. Indeed, in some cases there may not be a separate docking station and functionality attributed thereto herein can simply be integrated into the gas sampling device.

The reading device 432 can also be configured to both baseline data and sample data from the disposable sensor test strip 440 - where the term "baseline data" is defined as data collected before exposure of the disposable sensor test strip 440 to the gas sample or the patient or test subject and wherein the term "sample data" refers to data specific to the gas sample of the patient or test subject. The baseline data can reflect conditions of whatever gas happens to be in the testing chamber prior to obtaining a gas sample of a patient. Ambient air can purposefully be pushed through the testing chamber, and/or a particular reference gas sample of known composition can be put into the testing chamber for purposes of generating baseline data.

The docking station 430 is generally configured to be a docking location for the gas sampling device 410. The docking station 430 is generally configured to physically receive the gas sampling device 410. The docking station 430 can receive the gas sampling device 410 through a variety of structures and configurations that will be appreciated by those having ordinary skill in the art. The docking station 430 and the docking structure 421 of the gas sampling device 410 can have a mating configuration by which the docking station 430 receives the docking structure 421 of the gas sampling device 410. The docking station 430 and the docking structure 421 can define an interference fit. However, the docking station 430 can simply rest upon or in the docking structure 421. The docking station 430 and the docking structure 421 can be configured to position the disposable sensor test strip 440 and the reading device 432 in sufficient proximity to accommodate transmission of data between the reading device 432 and disposable sensor test strip 440. **In** some embodiments the docking station and the docking structure can be configured to position the disposable sensor test strip 440 and the reading device 432 within 6 cm, 5 cm, 4 cm, 3 cm, or 2 cm of each other, or even within 1 cm of each other.

The docking station 430 can have various additional components. The docking station 430 can have a processor 436 and memory 435. The processor 436 and memory 435 can be configured to process and store data obtained from the tested gas sample. For example, the memory 435 can store baseline data locally and the processor 436 can be configured to remove collected baseline data from the tested gas data to obtain adjusted data. Such adjusted data can remove some impact of the ambient environment on the tested gas data. The processor can be configured to compare the adjusted data (or, in some embodiments the tested gas data) to known data indicative of one or more diseases. Such a comparison can be used to identify the presence of a particular disease using a comparative algorithm. **In** yet another example, the processor of the docking station 430 can be configured to identify a defect in the disposable sensor test strip 440. Example defects can include manufacturing defects and/or premature exposure to ambient gases. The docking station 430 can be configured to collect, save, and potentially transmit records of such defects.

The docking station 430 can have networking hardware 434.

The networking hardware 434 can be configured to transmit data over a network to a remote system, including a cloud-based system. The remote system can be a hospital, clinic, laboratory, or other location. The networking hardware 434 can be configured to transmit data generated from testing the gas sample. The networking hardware 434 can be configured to transmit baseline data. The networking hardware can be configured to transmit adjusted data. The remote system can analyze the data it receives. For example, the remote system can be configured to compare the adjusted data to known data indicative of a plurality of diseases. That comparison can identify the presence of a particular disease.

The docking station 430 can have a user interface 438. The user interface 438 can be configured to communicate information to a user. For example, the user interface 438 can be configured to communicate an active data transmission, such as a data transmission between the docking station 430 and the gas sampling device 410 and/or between the docking station 430 and a network. The user interface 438 can be configured to communicate information about the current stage of the testing process, progress of the same, or what steps are next or what actions are required. For example, the user interface 438 can be configured to communicate that that the gas sampling device 410 is ready to receive a gas sample or that the docking station 430 has finished reading data from the gas sampling device 410. The user interface 438 can also be configured to communicate a defect in the sensor test strip. The user interface 438 can be configured to communicate through visual notification, audio notification, and the like. As a specific example, a flashing light can be used to indicate that the docking station 430 is transmitting data. The user interface 438 can include a light source such as an LED or similar light emitting device.

One example approach to using the system depicted in FIG. 4 will now be described. A disposable sensor test strip 440 is inserted into the gas sampling device 410 such that it is received by the gas testing chamber 426 defined by a housing of a gas sampling device. The gas sampling device 410 having the disposable sensor test strip 440 is docked to the docking station 430, and the reading device 432 of the docking station 430 reads baseline data from the disposable sensor test strip 440 through the housing 420 of the gas sampling device 410. The gas sampling device 410 is undocked from the docking station 430 after reading the baseline data, and a gas sample is received by the gas testing chamber such that the gas sample is brought into contact with the disposable sensor test strip 440. For example, the gas sampling device 410 may be physically grasped by a care provider and removed from the docking station 430 and physically handed to a patient or test subject who may then blow into the gas sampling device 410 to provide the gas sample to be analyzed. **In** other cases, the gas sampling device 410 may be held by the care provider instead of being held by the patient or test subject. The gas sampling device 410 can then be docked to the docking station 430 after receiving the gas sample, and the data from the tested gas is read from the disposable sensor test strip 440 by the reading device 432, wherein the adjusted data is read through the housing 420 of the gas sampling device 410. The disposable sensor test strip 440 can be configured to be single-use. As such, the disposable sensor test strip 440 can be disposed of following the collection of sample gas data from the disposable sensor test strip 440. Various other methods of using the system depicted in FIG. 4 are also contemplated.

The measurement zones 442 can include a plurality of discrete binding detectors in the form of graphene varactors that can include one or more analyte binding receptors bound thereto. All of the analyte binding receptors within a particular discrete binding detector can be the same with respect to their analyte binding properties, or at least some of the analyte binding receptors within a particular zone can be different from one another with respect to their analyte binding properties. Each discrete binding detector can be unique. Discrete binding detectors that are unique can be cross-reactive in that they bind to different portions or different configurations of the same chemical compound.

The plurality of graphene varactors can include those that are specific for different volatile organic compound. The plurality of graphene varactors can detect the presence of at least 5, 10, 15, 20, 30, 40 or more different volatile organic compounds. The number of different volatile organic compounds detected by the graphene varactors can be in a range wherein any of the forgoing numbers can serve as the upper or lower bound of the range provided that the upper bound is greater than the lower bound.

As referenced above, the capacitance of the graphene sensor can be measured by delivering an excitation current at a particular voltage and/or over a range of voltages. Measuring the capacitance provides data that reflects the binding status of analytes to the graphene sensor.

Various measurement circuitry can be used to measure the capacitance of the graphene sensor.

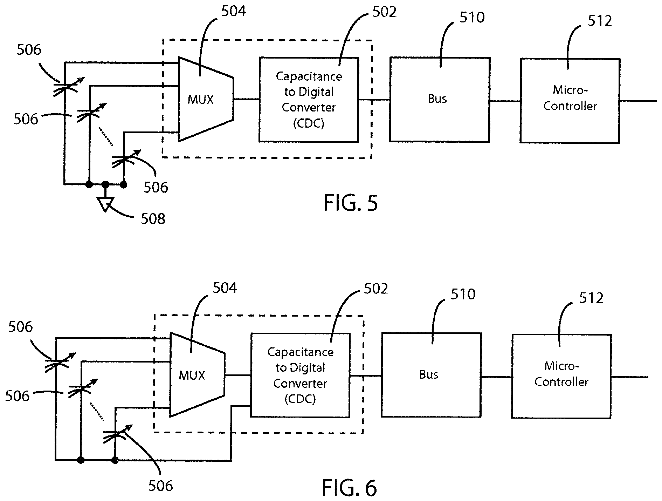

Referring now to FIG. 5, a schematic diagram is shown of circuitry to measure the capacitance of a plurality of graphene sensors. The circuitry includes a capacitance to digital converter (CDC) 502 in electrical communication with a multiplexor 504. The multiplexor 504 can provide selective electrical communication with a plurality of graphene varactors 506. The other side of the graphene varactors 506 can be in electrical communication with a ground 508.

The CDC 502 can be in electrical communication with a bus device 510. The bus device 510 can include various specific components, but can be selected from the group consisting of an I2C or an SPI. The bus device 510 can, in turn, be in electrical communication with a controller or processor.

For example, the bus device 510 can be in electrical communication with a microcontroller 512. The microcontroller 512 can, in turn, be in communication with other components of the device or system. It will be appreciated that not all of the components illustrated in FIG. 5 may be separate from one another. On the contrary, various components can be integrated together, including, but not limited to, the multiplexor 504, the CDC 502, and the bus device 510.

In operation, the excitation signal (an alternating voltage) is applied to the selected capacitor through the multiplexor and the electronic charge required to charge and discharge the graphene varactor is measured to determine the capacitance. The amplitude of the excitation signal is fixed by the CDC 502. The bias voltage at which the capacitance is measured is equal to the average voltage of the excitation signal. An exemplary CDC for this type of configuration includes Texas Instruments model FDC1004. The FDC1004 has an integrated multiplexor and IC. It will be appreciated that many other types of CDCs can also be used.

The graphene varactors may not be grounded. The other side of the graphene varactors can be connected to another device. For example, the other side of the graphene varactors can be connected to an independent excitation output of the CDC or another component. In this case, an excitation signal (again, an alternating voltage) is applied to all of the capacitors in the sensor array, and electronic charge required to charge and discharge the selected graphene varactor is measured through the multiplexor to determine the capacitance. The amplitude of the excitation signal is set by the CDC. The bias voltage at which the capacitance is measured can be equal to the difference between the bias voltage at the CDC input (via the multiplexor, usually equal to VCC/2, where VCC is the supply voltage) and the average voltage of the excitation signal. Since the excitation voltage is typically centered about VCC/2 for this type of CDC, the bias voltage is typically fixed at zero volts.

Referring now to FIG. 6, a further schematic diagram is shown of circuitry to measure the capacitance of a plurality of graphene sensors in accordance with the present invention disclosure. The circuitry includes a capacitance to digital converter (CDC) 502 in electrical communication with a multiplexor 504. The multiplexor 504 can provide selective electrical communication with a plurality of graphene varactors 506. In this embodiment, the other side of the graphene varactors 506 can be in electrical communication with an independent excitation output of the CDC.

The change in capacitance of the graphene varactor based on analyte binding depends on the excitation voltage used to measure the capacitance. Because of this, a greater amount of information can be gathered by varying the voltage of the excitation signal across a range of voltages. Various techniques can be used to achieve this. A pair of programmable digital to analog converters with different output voltages can be used in combination with a switch in order to achieve variation in the voltage of the excitation signal.

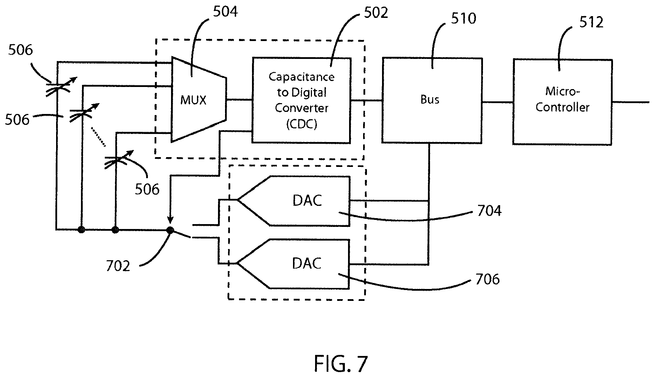

Referring now to FIG. 7, a further schematic diagram is shown of circuitry to measure the capacitance of a plurality of graphene sensors in accordance with the present invention disclosure. The circuitry includes a capacitance to digital converter (CDC) 502 in electrical communication with a multiplexor 504. The multiplexor 504 can provide selective electrical communication with a plurality of graphene varactors 506. The connection to the other side of the graphene varactors 506 can be controlled by a switch 702 (as controlled by the CDC) and can provide selective electrical communication with a first digital to analog converter (DAC) 704 and a second digital to analog converter (DAC) 706. The other side of the DACs 704, 706 can be connected to a bus device 510, or in some cases, the CDC 502.

In this case, the excitation signal from the CDC controls the switch between the output voltages of the two programmable Digital to Analog Converters (DACs). The programmed voltage difference between the DACs determines the excitation amplitude, providing an additional programmable scale factor to the measurement and allowing measurement of a wider range of capacitances than specified by the CDC. The bias voltage at which the capacitance is measured is equal to the difference between the bias voltage at the CDC input (via the multiplexor, usually equal to VCC/2, where VCC is the supply voltage) and the average voltage of the excitation signal, which is programmable. Buffer or amplifiers and/or bypass capacitance can be used at the DAC outputs to maintain stable voltages during switching. Many different ranges of DC bias voltages can be used. In some embodiments, the range of DC bias voltages can be from - 3 V to 3 V; -2.5 V to 2.5 V; -2 V to 2 V; - 1.5 V to 1.5 V; - 1 V to 1 V; or from -0.5 V to 0.5 V.

Many different aspects can be calculated based on the capacitance data. For example, aspects that can be calculated include maximum slope of capacitance to voltage, change in maximum slope of capacitance to voltage over a baseline value, minimum slope of capacitance to voltage, change in minimum slope of capacitance to voltage over a baseline value, minimum capacitance, change in minimum capacitance over a baseline value, voltage at minimum capacitance (Dirac point), change in voltage at minimum capacitance, maximum capacitance, change in maximum capacitance, ratio of maximum capacitance to minimum capacitance, response time constants, and ratios of any of the foregoing between different graphene sensors and particularly between different graphene sensors having specificity for different analytes.

The above calculated aspects can be used for various diagnostic purposes. In some cases, the above calculated aspects can be indicative of the identity and/or concentrations of specific volatile organic components of a gas sample. As such, each of the calculated values above can serve as a distinct piece of data that forms part of a pattern for a given patient and/or given gas sample. As also described elsewhere herein, the pattern can then be matched against pre-existing patterns, or patterns identified in real- time, derived from large stored data sets through techniques such as machine learning or other techniques, wherein such patterns are determined to be characteristic of various conditions or disease states. The above calculated aspects can also be put to other purposes, diagnostic and otherwise.

In some cases calculations, such as those described above, can be performed by a controller circuit. The controller circuit can be configured to receive an electrical signal reflecting the capacitance of the graphene varactors. The controller circuit can include a microcontroller to perform these calculations. The controller circuit can include a microprocessor in electrical communication with the measurement circuit. The microprocessor system can include components such as an address bus, a data bus, a control bus, a clock, a CPU, a processing device, an address decoder, RAM, ROM and the like. The controller circuit can include a calculation circuit (such as an application specific integrated circuit - ASIC) in electrical communication with the measurement circuit.

In addition, the system can include a non-volatile memory where sensitivity calibration information for the particular sensor is stored. By way of example, the sensor could be tested in a production facility, where its sensitivity to various analytes such as VOC's can be determined and then stored on an EPROM or similar component. In addition or alternatively, sensitivity calibration information can be stored in a central database and referenced with a sensor serial number when patient data is sent to a central location for analysis and diagnosis. These components can be included with any of the pieces of hardware described herein.

Components can be configured to communicate over a network, such as the internet or a similar network. A central storage and data processing facility can be included. Data gathered from sensors in the presence of the patient (local) can be sent to the central processing facility (remote) via the internet or a similar network, and the pattern from the particular patient being evaluated can be compared to those of thousands or millions of other patients, many of whom have been previously diagnosed with various conditions and wherein such condition data has been stored. Pattern matching algorithms can be used to find other patients or classes of patients (for example disease or condition specific classes) to which the current patient's pattern is most similar. Each class of patients can include a predetermined likelihood of having a given condition or disease state. In this manner, after pattern matching a likelihood of having a given condition or disease state can be provided back across the data network to the facility where the patient is currently at.

Methods can include any of the approaches and/or operations described herein. A method can include measuring a capacitance of the graphene varactor over a range of DC bias voltages, comparing the measured capacitance at one or more DC bias voltages to one or more corresponding baseline capacitance values and determining the presence or absence of an analyte based on the comparison.

The method can further include determining one or more aspects including, but not limited to, maximum slope of capacitance to voltage, change in maximum slope of capacitance to voltage over a baseline value, minimum slope of capacitance to voltage, change in minimum slope of capacitance to voltage over a baseline value, minimum capacitance, change in minimum capacitance over a baseline value and voltage at minimum capacitance (Dirac point), change in voltage at minimum capacitance, maximum capacitance, change in maximum capacitance, ratio of maximum capacitance to minimum capacitance, response time constants, and ratios of any of the foregoing between different graphene sensors and particularly between different graphene sensors having specificity for different analytes.

The method can include forming a capacitance to voltage curve from the measured capacitance of the graphene varactor over the range of DC bias voltages. The method can include comparing the formed capacitance to voltage curve with a baseline capacitance to voltage curve. The method can include distinguishing between different analytes binding to the graphene varactor. The method can further include measuring a capacitance of the graphene varactor over a range of DC bias voltages. The range of DC bias voltages can be from - 3 V to 3 V; -2.5 V to 2.5 V; -2 V to 2 V; - 1.5 V to 1.5 V; - 1 V to 1 V; -0.5 V to 0.5 V.

The patient can be prompted to take a breath or gas test (where the test could be performed either in a non-clinical setting such as their home or where such a prompt could cause them to come to a clinical setting to take the test).

A pattern including such things as sleep patterns (e.g. wearable, implant or non-contact in-home sensor), physiological data (autonomic tone measures), body weight (such as weight automatically measured by a mat in the house), activity levels (e.g. mobile device, wearable, implant or non-contact in-home sensor), etc. can be assessed, such as using an algorithm, and if the results of those factors so indicate, then the system can inform the user that they should administer or get a breath or gas test to detect early signs of heart failure decompensation. If a positive result, or data trends are beyond a normal range for the individual patient, then the system can inform the patient to seek medical care for early intervention.

A pattern including things such as sleep patterns, autonomic tone, respiratory rate, respiratory sounds, activity levels, etc., can be used to recommend to the user that they should administer a breath test (or come to a clinic to get a breath test) to detect early signs of a COPD exacerbation or repeat exacerbation. If a positive result, or data trends beyond normal range for the individual patient, seek medical care and/or use prescribed pharmaceutical (e.g. bronchodilators, corticosteroids, etc.) for early intervention.

Beyond, heart failure decompensation and COPD, such patterns and prompts to the patient to get a breath test can also be used for diabetes management and inflammatory bowel diseases (also including data regarding dietary intake, autonomic tone, etc. in the pattern) to detect early signs of a flare-up.

### Example 1 (not falling within the scope of the invention):

### Capacitance for a Graphene Varactor over a Range of Voltages

A graphene varactor was manufactured consistent with that described in U.S.Publ. Appl. No. 2014/0145735.

A baseline capacitance to voltage curve was established by measuring capacitance over a range of excitation voltages while flushing the sensor with nitrogen gas using an LCR meter. The DC bias voltage ranged from -0.5 V to 0.5 V. Using the same LCR meter, capacitance values over the same bias voltage range were also measured while flushing the sensor with a combination of nitrogen gas and octanol vapor. The total flow rate was 0.5 liters per minute in each experiment, octanol concentration was approximately 3.6 parts per million in nitrogen, sensors were exposed to the nitrogen gas and octanol vapor mixture for approximately 5 minutes before measurement for equilibration, the AC excitation signal had amplitude 50 mV and frequency 500 kHz.

As shown in FIG. 8, it can be seen that binding of octanol caused the capacitance to voltage curve to retain the same overall shape but be shifted to the right (e.g., the minimum capacitance occurred at a higher DC bias voltage).

### Example 2 (not falling within the scope of the invention)

### Capacitance for a Graphene Varactor over a Range of Voltages

A graphene varactor was prepared as described in Example 1. A baseline capacitance to voltage curve was established by measuring capacitance over a range of excitation voltages using an LCR meter as described above in Example 1. The DC bias voltage ranged from -1 V to 1 V. Using the same LCR meter, capacitance values over the same bias voltage range were also measured while flushing the sensor with a combination of nitrogen gas and acetone vapor. Details were the same as descried above in Example 1, except that the acetone concentration was approximately 25,000 parts per million.

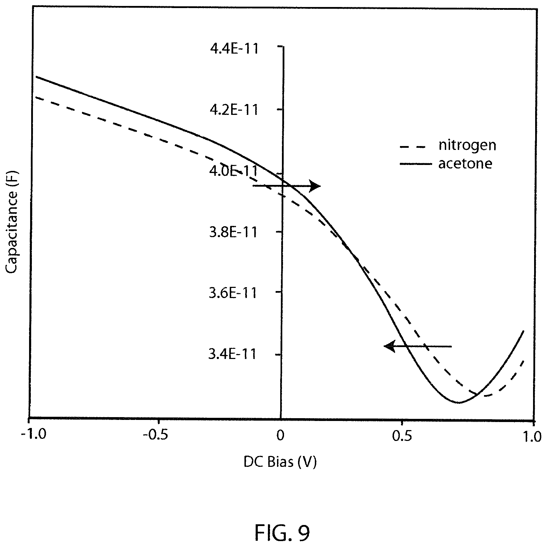

As shown in FIG. 9, it can be seen that binding of acetone caused the capacitance to voltage curve to change shape in that the top portion of the curve was shifted to the right, but the bottom portion of the curve was shifted to the left.

### Example 3 (not falling within the scope of the invention):

### Capacitance for a Functionalized Graphene Varactor over a Range of Voltages

A graphene varactor was prepared as described in Example 1. The graphene was then functionalized with pyrene-acetic acid by soaking the varactor in a 1 mM solution of pyrene acetic-acid in ethanol for 14 hours.

A baseline capacitance to voltage curve was established by measuring capacitance over a range of excitation voltages using an LCR meter as described above in Example 1.

The DC bias voltage ranged from -1 V to 1 V. Using the same LCR meter, capacitance values over the same bias voltage range were also measured while flushing the sensor with:
- a combination of nitrogen gas and gaseous acetone;
- a combination of nitrogen gas and gaseous octane; and
- a combination of nitrogen gas and gaseous octanol.

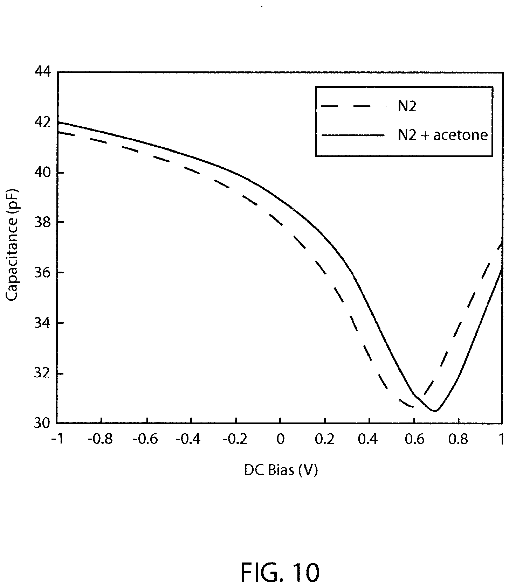

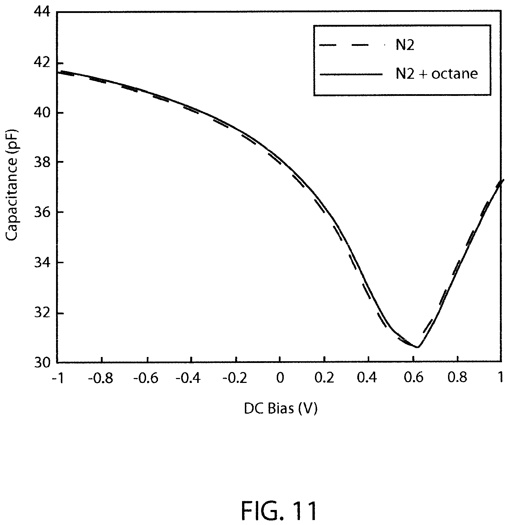

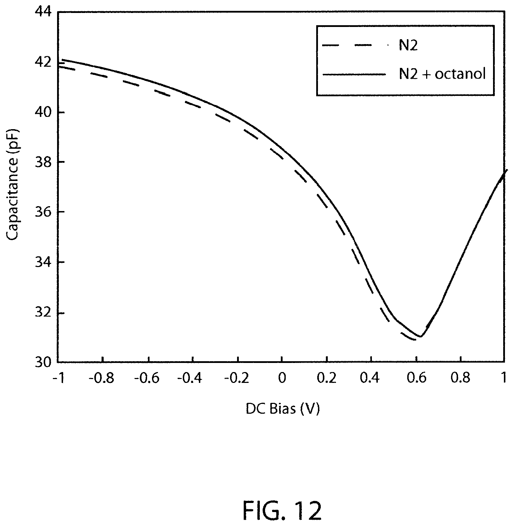

As shown in FIG. 10, it can be seen that binding of acetone caused the capacitance to voltage curve to shift sharply to the right, with the Dirac point increasing by approximately 0.1 volts. As shown in FIG. 11, it can be seen that binding of octane caused relatively small changes in the capacitance to voltage curve, but the shift to the right made the binding detectable. As shown in FIG. 12, it can be seen that binding of octanol caused relatively small changes in the capacitance to voltage curve, but the shift to the right made the binding detectable.

It should be noted that, as used in this specification and the appended clauses, the singular forms "a", "an", and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It should also be noted that, as used in this specification and the appended clauses, the phrase "configured" describes a system, apparatus, or other structure that is constructed or configured to perform a particular task or adopt a particular configuration to. The phrase "configured" can be used interchangeably with other similar phrases such as arranged and configured, constructed and arranged, constructed, manufactured and arranged, and the like. "Circuitry" can include both hardwired circuitry for execution of particular operations as well as processors that are programmed to execute instructions to provide the same functionality.

All publications and patent applications in this specification are indicative of the level of ordinary skill in the art to which this specification pertains.

## Claims

1. A system for measuring the presence of a plurality of different analytes on a plurality of graphene varactors (506) by evaluating capacitance across a range of DC bias voltages, comprising:
- a capacitance to digital converter, CDC (502);
- a plurality of graphene varactors (506) including graphene varactors with specificity for different gaseous analytes;
- a multiplexor (504) configured to selectively provide electrical communication between the CDC (502) and a plurality of the graphene varactors (506);
and
- a controller circuit configured to receive an electrical signal reflecting the capacitance of the graphene varactors (506) at a plurality of DC bias voltages;
wherein the CDC (502) is in electrical communication with the graphene varactors (506) and is configured to measure the capacitance of the graphene varactors (506) in response to an excitation signal over the range of DC bias voltages, wherein the measured capacitance across the range of DC bias voltages forms part of a pattern for a given sample;
wherein an excitation signal is applied to a selected graphene varactor (506) through the multiplexor (504); and
wherein the controller circuit is configured to record the measured capacitance values across the range of DC bias voltages.

2. The system of claim 1, wherein the excitation signal comprises an alternating voltage, and/or wherein the amplitude of the excitation signal is fixed by the CDC (502).

3. The system of claim 1, further comprising an electronic component in electrical communication with the controller circuit and the CDC (502), the electronic component selected from the group consisting of an I2C or an SPI.

4. The system of any of claims 1-3, wherein the graphene varactors (506) comprise a functionalized graphene varactor.

5. The system of any of claims 1-4, wherein the controller circuit is configured to calculate at least one parameter for the graphene varactors (506), the at least one parameter selected from the group consisting of:
- maximum slope of capacitance to voltage;
- change in maximum slope of capacitance to voltage over a baseline value;
- minimum slope of capacitance to voltage;
- change in minimum slope of capacitance to voltage over a baseline value; - minimum capacitance;
- change in minimum capacitance over a baseline value;
- voltage at minimum capacitance;
- change in voltage at minimum capacitance;
- maximum capacitance;
- change in maximum capacitance;
- ratio of maximum capacitance to minimum capacitance;
- response time constant; and
- ratios of any of the foregoing between the graphene varactor (506) and a second graphene varactor.

6. The system of any of claims 1-5, wherein the graphene varactors (506) are in electrical communication with an independent excitation output of the CDC (502), and/or further comprising one or more programmable digital to analog converters (DACs) (704, 706) in series with the graphene varactors (506), and/or further comprising a switch, the CDC (502) in electrical communication with the switch and controlling the switch to selectively provide communication with output voltages of two programmable digital to analog converters (DACs) (704, 706), the programmed voltage difference between the DACs determining the excitation amplitude, and the difference between the programmed average voltage of the DACs and the bias at the CDC (502) input determining the DC bias.

7. The system of any of the preceding claims 4-6 wherein the controller circuit comprises a microcontroller (512).

8. The system of any of the preceding claims wherein the graphene varactors (506) are grounded.

9. A gas sampling device (410), for example a hand-held breath sampling device or a gas sampling device (410) for use with catheters and/or endoscopy systems, for sensing gaseous analytes for example volatile organic compounds, the device comprising,
or incorporated in, a system according to any of the preceding claims.

10. A method for measuring the presence of a plurality of different analytes on a plurality of graphene varactors (506) by evaluating
capacitance across a range of DC bias voltages, comprising:
- selectively providing electrical communication between a capacitance to digital converter, CDC (502), and a plurality of graphene varactors (506) by means of a multiplexor (504), the plurality of graphene varactors (506) including graphene varactors with specificity for different gaseous analytes;
- measuring a capacitance of the plurality of the graphene varactors (506) in response to an excitation signal over a range of DC bias voltages using the CDC (502), wherein an excitation signal is applied to a selected graphene varactor (506) through the multiplexor (504);
- evaluating the measured capacitance values across a range of DC bias voltages to identify patterns associated with the presence, absence or amount of the gaseous analytes.

11. The method of claim 10, further comprising
determining one or more aspects selected from the group consisting of:
- maximum slope of capacitance to voltage;
- change in maximum slope of capacitance to voltage over a baseline value;
- minimum slope of capacitance to voltage;
- change in minimum slope of capacitance to voltage over a baseline value;
- minimum capacitance;
- change in minimum capacitance over a baseline value;
- voltage at minimum capacitance;
- change in voltage at minimum capacitance;
- maximum capacitance;
- change in maximum capacitance;
- ratio of maximum capacitance to minimum capacitance;
- response time constant; and
- ratios of any of the foregoing between two different graphene varactors, and/or further
comprising forming a capacitance to voltage curve from the measured capacitance of the graphene varactor (506) over the plurality of DC bias voltages, preferably further comprising comparing the formed capacitance to voltage curve with a baseline capacitance to voltage curve; and/or wherein the range of DC bias voltages is in the following ranges : - 3 V to 3 V; -2.5 V to 2.5 V; -2 V to 2 V; - 1.5 V to 1.5 V; - 1 V to 1 V; -0.5 V to 0.5 V.

12. Use of a system according to any of the claims 1-8, for determining the presence of a chemical analyte such as a volatile organic compound, preferably for measuring the chemical analyte.

13. Use of a device according to claim 9, for determining the presence of a chemical analyte such as a volatile organic compound, preferably for measuring the chemical analyte.

14. Use of a system and/or a device according to any of the claims 1-8, respectively 9, in a method according to claim 10 or 11.

## Patentansprüche

1. System zum Messen des Vorhandenseins mehrerer verschiedener Analyte auf mehreren Graphen-Varaktoren (506) durch Auswerten der Kapazität über einen Bereich von Gleichstromvorspannungen, umfassend:
- einen Kapazitäts-Digital-Wandler, CDC (502);
- mehrere Graphen-Varaktoren (506), die Graphen-Varaktoren mit Spezifität für verschiedene gasförmige Analyten umfassen;
- einen Multiplexer (504), der dazu ausgelegt ist, selektiv eine elektrische Verbindung zwischen dem CDC (502) und mehreren der Graphen-Varaktoren (506) herzustellen; und
- eine Steuerschaltung, die dazu ausgelegt ist, ein elektrisches Signal zu empfangen, das die Kapazität der Graphen-Varaktoren (506) bei mehreren Gleichstromvorspannungen widerspiegelt;
wobei der CDC (502) in elektrischer Verbindung mit den Graphen-Varaktoren (506) steht und dazu ausgelegt ist, die Kapazität der Graphen-Varaktoren (506) als Reaktion auf ein Anregungssignal über den Bereich von Gleichstromvorspannungen zu messen, wobei die gemessene Kapazität über den Bereich von Gleichstromvorspannungen einen Teil eines Musters für eine gegebene Probe bildet;
wobei über den Multiplexer (504) ein Anregungssignal an einen ausgewählten Graphen-Varaktor (506) angelegt wird; und
wobei die Steuerschaltung dazu ausgelegt ist, die gemessenen Kapazitätswerte über den Bereich der Gleichstromvorspannungen aufzuzeichnen.

2. System nach Anspruch 1, wobei das Anregungssignal eine Wechselspannung umfasst und/oder wobei die Amplitude des Anregungssignals durch den CDC (502) festgelegt ist.

3. System nach Anspruch 1, ferner umfassend ein elektronisches Bauteil, das in elektrischer Verbindung mit der Steuerschaltung und dem CDC (502) steht, wobei das elektronische Bauteil ausgewählt ist aus der Gruppe bestehend aus einem 12C oder einem SPI.

4. System nach einem der Ansprüche 1 bis 3, wobei die Graphen-Varaktoren (506) einen funktionalisierten Graphen-Varaktor umfassen.

5. System nach einem der Ansprüche 1 bis 4, wobei die Steuerschaltung dazu ausgelegt ist, zumindest einen Parameter für die Graphen-Varaktoren (506) zu berechnen, wobei der zumindest eine Parameter ausgewählt ist aus der Gruppe bestehend aus:
- maximaler Steigung von Kapazität zu Spannung;
- Änderung der maximalen Steigung von Kapazität zu Spannung gegenüber einem Basiswert;
- minimaler Steigung von Kapazität zu Spannung;
- Änderung der minimalen Steigung von Kapazität zu Spannung gegenüber einem Basiswert;
- minimaler Kapazität;
- Änderung der minimalen Kapazität gegenüber einem Basiswert;
- Spannung bei minimaler Kapazität;
- Änderung der Spannung bei minimaler Kapazität;
- maximaler Kapazität;
- Änderung der maximalen Kapazität;
- Verhältnis der maximalen Kapazität zur minimalen Kapazität;
- Ansprechzeitkonstante; und
- Verhältnissen eines der vorgenannten Parameter zwischen dem Graphen-Varaktor (506) und einem zweiten Graphen-Varaktor.

6. System nach einem der Ansprüche 1 bis 5, wobei die Graphen-Varaktoren (506) in elektrischer Verbindung mit einer unabhängigen Anregungsausgabe des CDC (502) stehen und/oder
ferner umfassend einen oder mehrere programmierbare Digital-AnalogWandler (DACs) (704, 706) in Reihe mit den Graphen-Varaktoren (506), und/oder
ferner umfassend einen Schalter, wobei der CDC (502) in elektrischer Verbindung mit dem Schalter steht und den Schalter ansteuert, um selektiv eine Verbindung mit Ausgangsspannungen von zwei programmierbaren Digital-Analog-Wandlern (DACs) (704, 706) herzustellen, wobei die programmierte Spannungsdifferenz zwischen den DACs die Anregungsamplitude bestimmt und wobei die Differenz zwischen der programmierten Durchschnittsspannung der DACs und der Vorspannung am Eingang des CDC (502) die Gleichstromvorspannung bestimmt.

7. System nach einem der vorhergehenden Ansprüche 4 bis 6, wobei die Steuerschaltung einen Mikrocontroller (512) umfasst.

8. System nach einem der vorhergehenden Ansprüche, wobei die Graphen-Varaktoren (506) geerdet sind.

9. Gasprobenentnahmevorrichtung (410), wie etwa handgehaltene Atemprobenentnahmevorrichtung oder Gasprobenentnahmevorrichtung (410) zur Verwendung mit Kathetern und/oder Endoskopiesystemen, zum Erfassen gasförmiger Analyten, wie etwa flüchtiger organischer Verbindungen, wobei die Vorrichtung ein System nach einem der vorhergehenden Ansprüche umfasst oder darin eingegliedert ist.

10. Verfahren zum Messen des Vorhandenseins mehrererverschiedener Analyte auf mehreren Graphen-Varaktoren (506) durch Auswerten der Kapazität über einen Bereich von Gleichstromvorspannungen, umfassend:
- selektives Bereitstellen einer elektrischen Verbindung zwischen einem Kapazitäts-Digital-Wandler, CDC (502), und mehreren Graphen-Varaktoren (506) mittels eines Multiplexers (504), wobei die mehreren Graphen-Varaktoren (506) Graphen-Varaktoren mit Spezifität für verschiedene gasförmige Analyten umfassen;
- Messen einer Kapazität der mehreren Graphen-Varaktoren (506) als Reaktion auf ein Anregungssignal über einen Bereich von Gleichstromvorspannungen unter Verwendung des CDC (502), wobei durch den Multiplexer (504) ein Anregungssignal an einen ausgewählten Graphen-Varaktor (506) angelegt wird;
- Auswerten der gemessenen Kapazitätswerte über einen Bereich von Gleichstromvorspannungen, um Muster zu erkennen, die dem Vorhandensein, dem Nichtvorhandensein oder der Menge von gasförmigen Analyten zugeordnet sind.

11. Verfahren nach Anspruch 10, ferner umfassend:
Bestimmen eines oder mehrerer Aspekte, die ausgewählt sind aus der Gruppe bestehend aus:
- maximaler Steigung von Kapazität zu Spannung;
- Änderung der maximalen Steigung von Kapazität zu Spannung gegenüber einem Basiswert;
- minimaler Steigung von Kapazität zu Spannung;
- Änderung der minimalen Steigung von Kapazität zu Spannung gegenüber einem Basiswert;
- minimaler Kapazität;
- Änderung der minimalen Kapazität gegenüber einem Basiswert;
- Spannung bei minimaler Kapazität;
- Änderung der Spannung bei minimaler Kapazität;
- maximaler Kapazität;
- Änderung der maximalen Kapazität;
- Verhältnis der maximalen Kapazität zur minimalen Kapazität;
- Ansprechzeitkonstante; und
- Verhältnissen eines der vorgenannten Parameter zwischen zwei verschiedenen Graphen-Varaktoren,
und/oder ferner umfassend das Bilden einer Kapazitäts-Spannungs-Kurve aus der gemessenen Kapazität des Graphen-Varaktors (506) über die mehreren Gleichstromvorspannungen, vorzugsweise ferner umfassend das Vergleichen der gebildeten Kapazitäts-Spannungs-Kurve mit einer Basiswert-Kapazitäts-Spannungs-Kurve;
und/oder wobei der Bereich der Gleichstromvorspannungen in den folgenden Bereichen liegt: -3 V bis 3 V; -2,5 V bis 2,5 V; -2 V bis 2 V; -1,5 V bis 1,5 V; -1 V bis 1 V; -0,5 V bis 0,5 V.

12. Verwendung eines Systems nach einem der Ansprüche 1 bis 8 zum Bestimmen des Vorhandenseins eines chemischen Analyten, wie einer flüchtigen organischen Verbindung, vorzugsweise zum Messen des chemischen Analyten.

13. Verwendung einer Vorrichtung nach Anspruch 9 zum Bestimmen des Vorhandenseins eines chemischen Analyten, wie einer flüchtigen organischen Verbindung, vorzugsweise zum Messen des chemischen Analyten.

14. Verwendung eines Systems und/oder einer Vorrichtung nach einem der Ansprüche 1 bis 8 bzw. nach Anspruch 9 in einem Verfahren nach Anspruch 10 oder 11.

## Revendications

1. Système de mesure de présence d'une pluralité de différents analytes sur une pluralité de varactors au graphène (506) par l'évaluation de la capacité sur une plage de tensions de polarisation en courant continu, CC, comprenant :
- un convertisseur capacité-numérique, CDC, (502) ;
- une pluralité de varactors au graphène (506) comportant des varactors au graphène ayant une spécificité pour différents analytes gazeux ;
- un multiplexeur (504) configuré pour fournir sélectivement une communication électrique entre le CDC (502) et une pluralité des varactors au graphène (506) ; et
- un circuit contrôleur configuré pour recevoir un signal électrique reflétant la capacité des varactors au graphène (506) à une pluralité de tensions de polarisation CC ;
dans lequel le CDC (502) est en communication électrique avec les varactors au graphène (506) et est configuré pour mesurer la capacité des varactors au graphène (506) en réponse à un signal d'excitation sur la plage de tensions de polarisation CC, dans lequel la capacité mesurée sur la plage de tensions de polarisation CC fait partie d'un motif pour un échantillon donné ;
dans lequel un signal d'excitation est appliqué à un varactor au graphène (506) sélectionné au travers du multiplexeur (504) ; et
dans lequel le circuit contrôleur est configuré pour enregistrer les valeurs de capacité mesurées sur la plage de tensions de polarisation CC.

2. Système selon la revendication 1, dans lequel le signal d'excitation comprend une tension alternative, et/ou dans lequel l'amplitude du signal d'excitation est fixée par le CDC (502).

3. Système selon la revendication 1, comprenant en outre un composant électronique en communication électrique avec le circuit contrôleur et le CDC (502), le composant électronique étant sélectionné parmi le groupe constitué d'un I2C ou d'un SPI.

4. Système selon l'une quelconque des revendications 1 à 3, dans lequel les varactors au graphène (506) comprennent un varactor au graphène fonctionnalisé.

5. Système selon l'une quelconque des revendications 1 à 4, dans lequel le circuit contrôleur est configuré pour calculer au moins un paramètre pour les varactors au graphène (506), l'au moins un paramètre étant sélectionné parmi le groupe constitué :
- d'une pente maximale de la capacité sur la tension ;
- d'une variation de la pente maximale de la capacité sur la tension par rapport à une valeur de base ;
- d'une pente minimale de la capacité sur la tension ;
- d'une variation de la pente minimale de la capacité sur la tension par rapport à une valeur de base ;
- d'une capacité minimale ;
- d'une variation de la capacité minimale par rapport à une valeur de base ;
- d'une tension à la capacité minimale ;
- d'une variation de la tension à la capacité minimale ;
- d'une capacité maximale ;
- d'une variation de la capacité maximale ;
- d'un rapport de la capacité maximale sur la capacité minimale ;
- d'une constante de temps de réponse ; et
- de rapports de l'un quelconque de ce qui précède entre le varactor au graphène (506) et un deuxième varactor au graphène.

6. Système selon l'une quelconque des revendications 1 à 5, dans lequel les varactors au graphène (506) sont en communication électrique avec une sortie d'excitation indépendante du CDC (502), et/ou comprenant en outre un ou plusieurs convertisseurs numériques-analogiques (DAC) programmables (704, 706) en série avec les varactors au graphène (506), et/ou comprenant en outre un commutateur, le CDC (502) étant en communication électrique avec le commutateur et commandant le commutateur pour fournir sélectivement une communication avec des tensions de sortie de deux convertisseurs numériques-analogiques (DAC) programmables (704, 706), la différence de tension programmée entre les DAC déterminant l'amplitude d'excitation, et la différence entre la tension moyenne programmée des DAC et la polarisation à l'entrée du CDC (502) déterminant la polarisation CC.

7. Système selon l'une quelconque des revendications précédentes 4 à 6 dans lequel le circuit contrôleur comprend un microcontrôleur (512).

8. Système selon l'une quelconque des revendications précédentes dans lequel les varactors au graphène (506) sont mis à la terre.

9. Dispositif d'échantillonnage de gaz (410), par exemple un dispositif d'échantillonnage d'haleine tenu à la main ou un dispositif d'échantillonnage de gaz (410) pour une utilisation avec des cathéters et/ou des systèmes d'endoscopie, pour détecter des analytes gazeux, par exemple des composés organiques volatils, le dispositif comprenant, ou étant incorporé dans, un système selon l'une quelconque des revendications précédentes.

10. Procédé de mesure de présence d'une pluralité de différents analytes sur une pluralité de varactors au graphène (506) par l'évaluation de la capacité sur une plage de tensions de polarisation en courant continu, CC, comprenant :
- la fourniture sélective d'une communication électrique entre un convertisseur capacité-numérique, CDC, (502) et une pluralité de varactors au graphène (506) au moyen d'un multiplexeur (504), la pluralité de varactors au graphène (506) comportant des varactors au graphène ayant une spécificité pour différents analytes gazeux ;
- la mesure d'une capacité de la pluralité des varactors au graphène (506) en réponse à un signal d'excitation sur une plage de tensions de polarisation CC en utilisant le CDC (502), dans lequel un signal d'excitation est appliqué à un varactor au graphène (506) sélectionné au travers du multiplexeur (504) ;
- l'évaluation des valeurs de capacité mesurées sur une plage de tensions de polarisation CC pour identifier des motifs associés à la présence, l'absence ou la quantité des analytes gazeux.

11. Procédé selon la revendication 10, comprenant en outre
la détermination d'un ou de plusieurs aspects sélectionnés parmi le groupe constitué :
- d'une pente maximale de la capacité sur la tension ;
- d'une variation de la pente maximale de la capacité sur la tension par rapport à une valeur de base ;
- d'une pente minimale de la capacité sur la tension ;
- d'une variation de la pente minimale de la capacité sur la tension par rapport à une valeur de base ;
- d'une capacité minimale ;
- d'une variation de la capacité minimale par rapport à une valeur de base ;
- d'une tension à la capacité minimale ;
- d'une variation de la tension à la capacité minimale ;
- d'une capacité maximale ;
- d'une variation de la capacité maximale ;
- d'un rapport de la capacité maximale sur la capacité minimale ;
- d'une constante de temps de réponse ; et
- de rapports de l'un quelconque de ce qui précède entre deux varactors au graphène différents, et/ou comprenant en outre la formation d'une courbe capacité sur tension à partir de la capacité mesurée du varactor au graphène (506) sur la pluralité de tensions de polarisation CC, comprenant en outre de préférence la comparaison de la courbe capacité sur tension formée avec une courbe capacité sur tension de base ; et/ou dans lequel la plage de tensions de polarisation CC est dans les plages suivantes : -3 V à 3 V ; -2,5 V à 2,5 V ; -2 V à 2 V ; -1,5 V à 1,5 V ; -1 V à 1 V ; -0,5 V à 0,5 V.

12. Utilisation d'un système selon l'une quelconque des revendications 1 à 8, pour déterminer la présence d'un analyte chimique tel qu'un composé organique volatil, de préférence pour mesurer l'analyte chimique.

13. Utilisation d'un dispositif selon la revendication 9, pour déterminer la présence d'un analyte chimique tel qu'un composé organique volatil, de préférence pour mesurer l'analyte chimique.

14. Utilisation d'un système et/ou d'un dispositif selon l'une quelconque des revendications 1 à 8, respectivement 9, dans un procédé selon la revendication 10 ou 11.
